# EUROPÄISCHE PATENTSCHRIFT

(11) **EP 4 164 705 B1**
(45) Veröffentlichungstag und Bekanntmachung des Hinweises auf die Patenterteilung: **02.10.2024**
(21) Anmeldenummer: 21763025.0
(22) Anmeldetag: 06.08.2021
(51) Int. Cl.: A61L 27/18, A61L 27/56, A61F 2/30, B29C 64/153

(54) **VERFAHREN ZUR HERSTELLUNG EINES BIOKOMPATIBLEN IMPLANTATS UND IMPLANTAT**
METHOD FOR PRODUCING A BIOCOMPATIBLE IMPLANT, AND IMPLANT
PROCÉDÉ DE FABRICATION D'UN IMPLANT BIOCOMPATIBLE ET IMPLANT CORRESPONDANT

(30) Priorität: 07.08.2020 DE 102020210038
(43) Veröffentlichungstag der Anmeldung: 19.04.2023
(73) Patentinhaber: Karl Leibinger Medizintechnik GmbH & Co. KG, 78570 Mühlheim/Donau (DE)
(72) Erfinder: AKSU, Adem, 78054 VS-Schwenningen (DE); REINAUER, Frank, 78576 Emmingen-Liptingen (DE); WOLFRAM, Tobias, 63303 Dreieich (DE)
(74) Vertreter: Isarpatent
(86) Internationale Anmeldenummer: PCT/EP2021/071975
(87) Internationale Veröffentlichungsnummer: WO 2022/029282

(56) Entgegenhaltungen:
- EP-A2- 3 308 747
- DE-A1- 102016 110 500
- DE-B3- 102016 110 501

## Beschreibung

Die vorliegende Erfindung betrifft ein Verfahren zur Herstellung eines biokompatiblen Implantats.

Die DE 10 2016 110 501 B3 offenbart ein Verfahren zum Herstellen eines Gegenstandes zur chirurgischen Anwandung mit den Schritten: Bereitstellen eines Kunststoff-Pulvers, Erhitzen und Verpressen des Kunststoff-Pulvers unter Ausbildung zumindest eines Zwischenstücks, mechanisches Zerkleinern des zumindest einen Zwischenstücks zu einem Granulat und Verbinden des Granulats zu einem einstückigen Grundkörper.

Die EP 3 308 747 A offenbart ein Verfahren zur Herstellung einer Prothese mit einer inneren Schicht aus Polyaryletherketon, einer an die innere Schicht angrenzenden ersten äußeren Schicht aus porösem Polyaryletherketon und einer zweiten äußeren Schicht aus porösem Polyaryletherketone, wobei die Anzahl der Poren in den äußeren Schichten unterschiedlich sein kann.

Polyaryletherketone (PAEK) sind Hochleistungskunststoffe, welche Polymerkette Ether- und Ketongruppen sowie Phenylen-Einheiten aufweisen. Sie haben gute mechanische Eigenschaften auch bei höheren Temperaturen und bei wechselnder Beanspruchung sowie hinsichtlich ihres Verschleißverhaltens. Außerdem zeigen sie als biokompatibles Implantatmaterial eine sehr gute Verträglichkeit, so dass sie bereits seit längerem Verwendung bei der Herstellung von Implantaten gefunden haben. Die DE 10 2016 110 501 B3 offenbart ein additives Herstellungsverfahren für Implantate, mittels einem Lasersinterverfahren aus einem Granulat hergestellt werden.

Für eine erfolgreiche Integration und Fixierung eines Implantates stellt die Neubildung von Blutgefäßen einen entscheidenden Prozess dar. Jedoch ist bei den bisher bekannten Kunststoffen "aus Polyaryletherketonen ein Einwachsen von Weichgewebe und Knochen nicht definiert möglich.

Es ist daher die Aufgabe der vorliegenden Erfindung, ein Verfahren zur Herstellung eines Implantats sowie ein entsprechendes Implantat anzugeben, das das Einwachsen von Weichgewebe und Knochen besser ermöglicht und das eine erhöhte Festigkeit bietet.

Diese Aufgabe wird durch das Verfahren mit den Merkmalen des Anspruchs 1 gelöst.

Erfindungsgemäß ist das betreffende Verfahren mit folgenden Schritten vorgesehen:
S1) Bereitstellen eines Kunststoff-Pulvers aus einem Polyaryletherketon;
S2) Erhitzen und Verpressen des Kunststoff-Pulvers unter Ausbildung zumindest eines Zwischenstücks;
S3) mechanisches Zerkleinern des zumindest einen Zwischenstücks zu einem Granulat; und
S4) thermisches Verbinden des Granulats in einem Formwerkzeug zu einem Implantat, wobei das Granulat beim thermischen Verbinden mit einer räumlich inhomogenen Wärmeverteilung beaufschlagt wird, wobei das Implantat einen Implantatkörper mit wenigstens einem ersten porös ausgebildeten Teilabschnitt und einem zweiten porös ausgebildeten Teilabschnitt aufweist, wobei sich der erste poröse Teilabschnitt und der zweite poröse Teilabschnitt hinsichtlich ihrer Porosität unterscheiden.

Mittels des thermischen Verbindens unter Beaufschlagung mit einer inhomogenen Wärmeverteilung können also individuelle dreidimensionale Implantate aus einem Polyaryetherketon-Kunststoff mit einer partikulären Formulierung aus wenigstens einem Granulat mit einer Mehrzahl poröser Teilabschnitte, die sich hinsichtlich ihrer Porosität unterscheidenden, hergestellt werden. Es sind selbstverständlich auch Implantate mit mehreren thermisch verbundenen Granulaten erfindungsgemäß herstellbar.

Das poröse und topographisch individuell definierte Implantat als Verfahrensprodukt ermöglicht umgebungsspezifisch ein verbessertes Einwachsen von Weichgewebe und Knochen. Dieses umfassende vaskuläre Einwachsen hilft dabei, wichtige Zellen, die Infektionen bekämpfen und damit in der Lage sind, die Inflammation zu modifizieren, tief in das Implantat zu transportieren. Zugleich erhöht das Einwachsen von biologischem Gewebe die Festigkeit des Implantats. Das Implantat wird primär spezifisch über die porösen Eigenschaften sowie über die makrostrukturellen Eigenschaften des Herstellungsprozesses fixiert. Das Implantat kann daher entsprechend als sowohl mikro- als auch makro strukturiert angesehen werden, es wird nicht schichtweise aufgebaut, sondern als einstückiges Bauteil gefertigt.

Vorteilhafte Ausführungsformen und Varianten finden sich in den Unteransprüchen.

Bei einer vorteilhaften Variante des erfindungsgemäßen Verfahrens, die in einfacher Weise eine Ausdifferenzierung des Produkts gestattet, ist das Formwerkzeug mit einer Mehrzahl von Heizeinrichtungen heizbar, die bei Verbinden des Granulats jeweils separat angesteuert werden, um die inhomogene Wärmeverteilung zu erzeugen. Dabei können die Heizeinrichtungen beispielsweise in verschiedenen Raumrichtungen um das thermisch zu verbindende Granulat angeordnet sein. Weiter kann die Inhomogenität der Wärmeverteilung auch durch eine zeit- und leistungsabhängige Ansteuerung der Heizeinrichtungen realisiert werden.

In einer anderen vorteilhaften Ausführung ist das Polyaryletherketon ein Polyetheretherketon, bei dem jeweils zwei Ethergruppen und eine Ketongruppe aufeinander folgen, wodurch sich ein besonders gut verträgliches Implantat mit ausgewogenem Eigenschaftsprofil fertigen lässt.

Um an dem herzustellenden Implantat ein Optimum hinsichtlich Wärmeverteilung und -eintrag zu ermöglichen, kann das aus dem Zwischenstück zerkleinerte, Granulat bei einer zweckmäßigen Variante mit Partikeln mit einem runden, einem abgerundeten, einem teilweise eckigen oder einem mehreckigen Querschnitt gebildet werden. Unter rund ist in diesem Fall nicht nur ein kreisrunder Querschnitt zu verstehen, sondern auch beispielsweise ein ovaler oder ein elliptischer Querschnitt, oder auch ein unregelmäßig abgerundeter Querschnitt. Daneben kann der Querschnitt auch halbkreisförmig oder derartig vieleckig, dass er näherungsweise rund erscheint. Unter den kleinzahligen mehreckigen Querschnitten sind beispielsweise vier-, fünf-, sechs- oder achteckige Querschnitte denkbar. Weiter sind beispielsweise auch unterschiedliche Mischungen von Granulaten unterschiedlichen Querschnitts mit Blick auf Formgebung und Größe denkbar.

In einer bevorzugten Weiterbildung, bei der das Implantat mit seinen porösen Teilabschnitten eine gewisse Homogenität aufweist, kann das aus dem Zwischenstück zerkleinerte, wenigstens eine Granulat mit einem einheitlich runden Querschnitt zerkleinert werden. Die porösen Teilabschnitte des Implantats sind jedoch nicht zwingend homogen ausgebildet.

Eine andere bevorzugte Weiterbildung kann darin bestehen, das Kunststoff-Pulver vor der Herstellung des Granulats einer antimikrobiellen Behandlung zu unterziehen und/oder dem Kunststoff-Pulver vor der Herstellung des Granulats eine bioaktive Komponente beizumischen, so dass an dem Granulat und später dem Implantat eine intrinsisch antimikrobiell aktive Porenstruktur ausgebildet werden kann.

Um die Festigkeitscharakteristik des herzustellenden Implantats flexibel einstellen zu können, kann bei einer vorteilhaften Variante des Verfahrens die Partikelgröße des Granulats variiert werden, wobei das Granulat eine Partikelgröße zwischen 1 µm und 3000 µm aufweist. Das herzustellende Implantat kann beispielsweise mit einer Mehrzahl sich unterscheidender Schichten ausgebildet werden, die sowohl mit unporöser Struktur als auch mit porösen Strukturen ausgebildet werden können. Hierzu kann das Formwerkzeug in einer vorteilhaften Variante des Verfahrens vor dem Verbinden des wenigstens einen Granulats differentiell, also mit einer Mehrzahl sich hinsichtlich ihrer Partikelgröße und/oder -form unterscheidender Granulate, befüllt werden. Hierbei können die sich unterscheidenden Granulate beim thermischen Verbinden jeweils eine Schicht mit eigenständigen Eigenschaften bilden, wobei wiederum auch prinzipiell zwei oder mehrere Schichten aus dem gleichen ursprünglichen Granulat denkbar sind.

Aufgrund von Modifikationen der partikulären Ausprägung der Granulatformen sowie Beimischungen von bioaktiven Komponenten und der differentiellen Befüllung der Aufbauformen im Herstellungsprozess kann das Implantat auch eine strukturierte Porosität im Sinne einer vektoriellen Porenverteilung mit unterschiedlicher Bioaktivität aufweisen und so die biologische Fixierung des Implantats erreicht werden.

Zur weiteren Variation der Eigenschaften des Implantates kann bei einer anderen vorteilhaften Variante während des Verbindens des wenigstens einen Granulats seitens des Formwerkzeugs ein vorgebbarer Druck auf das wenigstens eine Granulat ausgeübt werden, wodurch beispielsweise die Scherbelastbarkeit des Implantats erhöht werden kann.

In einer bevorzugten Variante des Verfahrens kann das Formwerkzeug ein Formgebungsmittel aufweisen, durch welches beim thermischen Verbinden des Granulats innerhalb der Struktur des individuellen dreidimensionalen Implantats ein Hohlraum ausgebildet wird. Dabei wird also der makroskopische Aufbau des Implantatkörpers derart ausgestaltet, dass dieser als Hohlkammersystem zur Aufnahme bspw. von Hydrogelen oder Biotinten verwendet werden kann. Dadurch kommt dem Implantatkörper eine Depotwirkung zu.

Das Formgebungsmittel kann dabei eine Negativform des auszubildenden Hohlraumes sein, z.B. eine an dem Formwerkzeug ausgebildete Vorwölbung oder Ausbuchtung, von welcher das Implantat nachträglich entformt werden muss, auch ist eine ballonartige Struktur vorstellbar, die etwa mit einem Fluid befüllt ist und nach dem Verbinden des Granulats aus dem dann entstandenen Hohlraum wieder entfernt wird. Schließlich ist auch ein Formgebungsmittel in Art eines Rotationsformwerkzeugs denkbar.

Das Implantat wird während seiner Herstellung dreidimensional an eine Aufnahmestruktur im Körper eines menschlichen Körpers angepasst ist, wobei das angepasste Implantat an seinem Implantatkörper mit einem ersten porös ausgebildeten Teilabschnitt und mit einem zweiten porös ausgebildeten Teilabschnitt versehen ist, wobei sich die beiden porösen Teilabschnitte hinsichtlich ihrer Porosität unterscheiden. Durch die Ausbildung mit den porösen Teilabschnitten innerhalb seiner Struktur wird ein zumindest teilporöses Implantat mit optimierter Osteointegration und möglicher Bioaktivität etabliert. Die betreffenden Teilabschnitte des Implantats sind vorzugsweise mit einer interkonnektierenden Porenstuktur vorgesehen und vermittels der dadurch gebildeten offenen durchgängigen Hohlräume eingerichtet, dass Versorgungsstrukturen für neu zu bildende Knochenstruktur einwachsen können. Die Porosität kann auch nur partiell im Implantat eingebracht sein.

Aufgrund von dessen ausgewogenen Eigenschaften ist eine vorteilhafte Weiterbildung des Implantats aus Polyetheretherketon (PEEK) gebildet.

In einer vorteilhaften Gestaltung des Implantats, die eine Variation von dessen Eigenschaften gestattet, kann das Implantat einen schichtweisen Aufbau mit einer Mehrzahl von Schichten aufweisen, von welchen wenigstens eine die porösen Teilabschnitte aufweist. Beispielsweise kann etwa das Implantat mit einer massiven (unporösen) und einer (ggf. partiell) porösen Schicht mit den unterschiedlich porösen Teilabschnitten versehen sein. Es ist auch möglich, dass die porösen Teilabschnitte selbst jeweils eine solche Schicht bilden.

Weiter kann an dem Implantat z.B. eine dieses zumindest teilweise begrenzende Außenschicht vorgesehen sein, deren Struktur sich von einer oder mehreren Innenschichten unterscheidet, wobei die Außenschicht insbesondere massiv ausgebildet sein kann. Hierbei kann das Implantat weiter wenigstens eine an die Außenschicht grenzende innere Schicht aufweisen, die mit Teilabschnitten sich unterscheidender partieller Porosität versehen sein kann.

Bei einer vorteilhaften Gestaltung kann der Implantatkörper eine Gesamtporosität mit Wertebereichen zwischen 10% und 80% annehmen, insbesondere Teilabschnitte mit einer Porosität zwischen 10-20%, 20-40%, 40-60% und/oder 60-80% aufweisen, wodurch flexible Festigkeitscharakteristika an dem Implantat erreicht werden können.

Die porösen Teilabschnitte können dabei zweckmäßigerweise mit unterschiedlichen Porengrößen ausgebildet sein, wobei die Porengrößen in einem Bereich von 1 µm bis 3500 µm variieren können. Mit sich unterscheidenden Porengrößen kann ein Porositätsgradient gebildet werden, wodurch an dem Implantat die Zellmigrationseigenschaften und die Diffusion von Nährstoffen und Abfallprodukten an dem Implantat verbessert werden können. Der Gradient kann dabei eingerichtet sein, sich schichttreu oder über Schichten hinweg zu erstrecken und in einer Auftragung eine Kurve mit oder ohne Unstetigkeiten sein.

In einer vorteilhaften Gestaltung mit der zum Beispiel Heilungsprozesse besser unterstützt werden können, kann das Implantat innerhalb seiner Struktur wenigstens einen Hohlraum aufweisen, der zur Aufnahme einer bioaktiven Substanz eingerichtet ist.

Dabei kann die Substanz wahlweise vor oder nach dem Einbringen des Implantats in die Aufnahmestruktur des menschlichen Körpers in dem Hohlraum untergebracht werden. Auf diese Weise kann ein poröses Implantat mit Platzhalterstruktur in makroskopischer Ausführung zur Aufnahme etwa von anorganischen Calciumphosphat-basierten Materialien, die als injizierbare Substanz eingebracht werden können und die Geweberegenration verbessern, ausgebildet werden.

In einer vorteilhaften Weiterbildung kann das poröse patienten-spezifische Implantat, beispielsweise aus PEEK, mit Platzhalterstrukturen im Größenbereich von 500 µm bis 2 mm zur Aufnahme von bioaktiv wirksamen Hydrogel- und/oder Biotinten -Formulierungen zur Optimierung von zelltherapeutischen Applikationen versehen sein. Weiter kann das poröse Implantat die Eigenschaften des Hohlraums dahingehend nutzen, als "Biokäfig" (Biocage) für Zellkultursysteme zu dienen. Es sind aber auch andere Größen des Hohlraums denkbar.

Ion einer anderen Weiterbildung des Implantats kann der Hohlraum mit einem Schnittstellenabschnitt versehen sein, der den Hohlraum mit dem Außenraum des Implantats verbindet. Über den Schnittstellenabschnitt ist wenigstens eine Substanz in dem Hohlraum injizierbar, bevor oder nachdem das Implantat in die Aufnahmestruktur eingesetzt ist.

Zweckmäßigerweise kann bei noch einer andren Weiterbildung der Schnittstellenabschnitt mit einem Führungsmittel zur Anlage, Aufnahme oder Ausrichtung eines Injektionsmittels versehen sein. Zur Führung kann dabei der Schnittstellenabschnitt zum Beispiel zylindrisch ausgebildet sein, und an seinem Rand mit einer Stufe in Art eines Flansches zur Anlage und Ausrichtung des Injektionsmittels versehen sein. Es sind aber auch andere Ausgestaltungen denkbar.

In bevorzugten Weiterbildungen können die in dem Hohlraum zu bevorratenden Substanzen dem Implantat zugeordnet als wenigstens ein Hydrogel, wenigstens eine Biotinte, wenigstens ein anorganisches Calciumphosphat-basiertes Material oder als Zellkultur vorgesehen sein.

Ein verbessertes Anwachsen von Knochengewebe kann mittels einer vorteilhaften Gestaltung des Implantats erreicht werden, das an seiner Oberfläche zumindest einen angerauten Abschnitt aufweist. Eine derart aktive, weil stark vergrößerte Implantatoberfläche kann beispielsweise durch Sandstrahlen oder Säureätzen erreicht werden, da die erzeugte Mikrorauheit eine Einheilung des Implantates begünstigt.

Ähnliche Vorteile beim Anwachsen von Geweben weist dabei ein hydrophil ausgebildeter Oberflächenabschnitt auf. Andererseits kann ein hydrophob ausgebildeter Oberflächenabschnitt gegebenenfalls die Hafteigenschaften und damit die Anlagerung von Bakterien beeinflussen. In einer zweckmäßigen Weiterbildung kann daher das Implantat beispielsweise an einer ersten Seite zumindest bereichsweise hydrophob und auf einer zweiten Seite zumindest bereichsweise hydrophil ausgebildet sein.

Um das Implantat individuell an die Aufnahmestruktur anpassen zu können, kann in einer Weiterbildung das Implantat flexibel formbar vorgesehen sein, insbesondere durch Anwärmen in Kochsalzlösung flexibel formbar sein.

Die obigen Ausgestaltungen und Weiterbildungen lassen sich, sofern sinnvoll, beliebig miteinander kombinieren. Weitere mögliche Ausgestaltungen, Weiterbildungen und Implementierungen der Erfindung umfassen auch nicht explizit genannte Kombinationen von zuvor oder im Folgenden bezüglich der Ausführungsbeispiele beschriebenen Merkmalen der Erfindung. Insbesondere wird dabei der Fachmann auch Einzelaspekte als Verbesserungen oder Ergänzungen zu der jeweiligen Grundform der vorliegenden Erfindung hinzufügen.

Die Erfindung wird nachstehend anhand von Ausführungsbeispielen in den Figuren der Zeichnung näher erläutert.

Es zeigen:
- Fig. 1: eine schematische Ablaufdarstellung einer Ausführungsform des erfindungsgemäßen Verfahrens zur Herstellung des Implantats mit den Verfahrensschritte S1 bis S4;
- Fig. 2: eine Querschnittsansicht eines in der Befüllung mit Granulat befindlichen, geöffneten Formwerkzeugs;
- Fig. 3: eine Seitenansicht des Formwerkzeugs gemäß Fig. 2 in geschlossenem Zustand nach der Herstellung des Implantatkörpers;
- Fig. 4: eine geschnittene Seitenansicht eines Implantatkörpers mit einer Mehrzahl von Schichten und unterschiedlich porös ausgebildeten Teilabschnitten;
- Fig. 5: eine geschnittene Seitenansicht eines Implantatkörpers mit massiver Außenschicht;
- Fig. 6: eine geschnittene Seitenansicht eines Implantatkörpers in dessen Struktur ein Hohlraum ausgebildet ist;
- Fig. 7: eine geschnittene Seitenansicht eines Teilausschnitt der Gestaltung gemäß Fig. 6;
- Fig. 8: eine geschnittene Seitenansicht einer Gestaltung des Implantatkörpers des Implantates mit poröser Oberfläche und porösen Teilabschnitten aus einem sechseckigen Granulat mit Beimischungen; und
- Fig. 9: eine geschnittene Seitenansicht einer Gestaltung des Implantatkörpers des Implantates mit poröser Oberfläche und porösen Teilabschnitten aus einem fünfeckigen Granulat mit Beimischungen.

In allen Figuren sind gleiche bzw. funktionsgleiche Elemente und Vorrichtungen - sofern nichts anderes angegeben ist - mit denselben Bezugszeichen versehen worden.

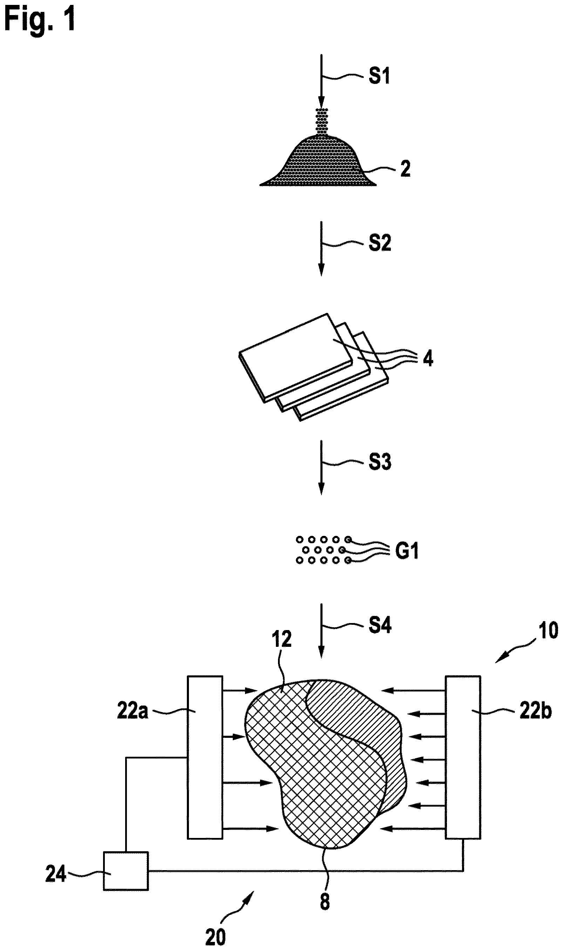

Fig. 1 zeigt eine schematische Ablaufdarstellung einer Ausführungsform des erfindungsgemäßen Verfahrens zur Herstellung des Implantats mit den Verfahrensschritte S1 bis S4.

Gemäß Fig. 1 wird zunächst ein Kunststoff-Pulver 2 in Form eines PEEK-Pulvers 2 bereitgestellt (Schritt S1). Das rieselfähige Kunststoff-Pulver 2 wird im Anschluss daran, durch den Schritt S2 gekennzeichnet, mittels eines sinterähnlichen Verfahrens verpresst. Dabei entstehen ein oder mehrere einstückige / in sich zusammenhängende Zwischenstücke 4. Insbesondere werden die Zwischenstücke 4 durch ein Verpressen mit gleichzeitigem Erhitzen des Kunststoff-Pulvers 2 erreicht, wobei die Zwischenstücke 4 letztendlich rechteckförmige Platten ausbilden. Die Temperatur der Zwischenstücke 4 bei diesem Sintern/Urformen der Zwischenstücke 3 ist stets unterhalb der Zersetzungstemperatur des verwendeten Kunststoff-Pulvers 2 (bei mehreren Kunststoffmaterialien unterhalb der Zersetzungstemperatur der am niedrig schmelzenden Materialkomponente des verwendeten Kunststoff-Pulvers 2). Bevorzugt ist zur Herstellung des jeweiligen Zwischenstückes 4 eine Negativformen vorhanden, in die das Kunststoff-Pulver 2 zunächst eingefüllt wird und im Anschluss daran aufgeheizt sowie, mit einer Presskraft beaufschlagt, zusammengepresst wird, sodass sich ein in sich festes Gebilde in Form der Zwischenstücke 4 bildet.

Im Anschluss an das Fertigen der Zwischenstücke 4 wird gemäß Schritt S3 jedes Zwischenstück 4 wieder definiert zerkleinert. Die Zwischenstücke 3 werden in Partikel unter Ausbildung eines Granulats G1 zerkleinert. Die Partikel des Granulats G1 weisen eine im Wesentlichen einheitliche Form auf, die durch die konkrete Ausführung der mechanischen Zerkleinerung ausgeführt wird. In diesem Ausführungsbeispiel werden runde Partikel, in Form von kugelartigen oder im Querschnitt ovalen Partikeln erzeugt.

Gemäß Schritt S4 folgt im Anschluss daran ein thermisches Verbinden des in seiner Form eingestellten Granulats 4 zu dem einteiligen individuellen dreidimensionalen Implantatkörper 8. In diesem Ausführungsbeispiel dient zum thermischen Verbinden die Beaufschlagung mit einer räumlich inhomogenen Wärmeverteilung seitens eines Formwerkzeugs 20, so dass sich an dem Implantatkörper 8 zwei poröse Teilabschnitte 12, 13 unterschiedlicher Porosität ausbilden (unter Verwendung eines hier nicht dargestellten Formwerkzeuges).

Der Implantatkörper 8 weist in diesem Verfahren nach Durchführen des Schrittes S4 im Wesentlichen die fertige Form des herzustellenden Implantates 10 auf. Das Implantat 10 ist hierbei auf typische Weise als ein Implantat 1 zur Osteosynthese bzw. Frakturversorgung ausgebildet. Das thermische Verbinden wird derart ausgeführt, dass das Implantat 10 / der Implantatkörper 8 eine poröse, vorzugsweise eine offenporige Struktur aufweist. Alternativ sind auch geschlossene porige Strukturen umsetzbar.

Weiter ist in der Fig. 1 zu erkennen, dass an dem Formwerkzeug 20 zwei Heizeinrichtungen 22a, 22b angeordnet sind, die durch eine Steuerung 24 betrieben werden. Die Inhomogenität der Wärmeverteilung, mit der das Granulat G1 beaufschlagt wird, wird durch die beiden separat angesteuerten Heizeinrichtungen 22a, 22b erreicht, wobei die Heizeinrichtung 22a Wärme einer Temperatur T1 abgibt und die Heizeinrichtung 22b Wärme einer Temperatur T2, so dass an dem Implantatkörper 8 Teilabschnitte 12, 13 mit unterschiedlicher Porosität ausgebildet werden.

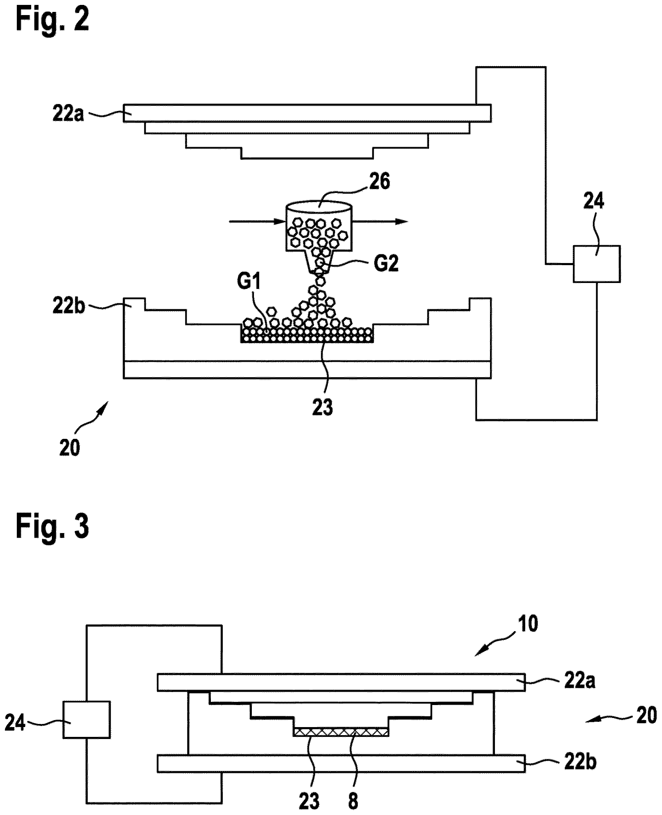

Fig. 2 zeigt eine Querschnittsansicht eines in der Befüllung mit Granulat befindlichen, geöffneten Formwerkzeugs.

Gemäß Fig. 2 wird das Formwerkzeug 20 zur Durchführung des erfindungsgemäßen Verfahrens mit zwei Granulaten G1, G2 durch einen Befüller 26 befüllt wird. Das erste Granulat G1 mit einem runden Querschnitt ist bereits in dem an der unteren Heizeinrichtung 22b angeordneten Formbett 23 angeordnet, und der Befüller 26 befüllt das Formbett 23 nun differentiell mit einem weiteren Granulat G2, das einen abweichenden Querschnitt in der Granulatform aufweist, nämlich einen sechseckigen Querschnitt.

Fig. 3 zeigt eine Seitenansicht des Formwerkzeugs gemäß Fig. 2 in geschlossenem Zustand nach der Herstellung des Implantatkörpers.

In der Fig. 3 erkennt man, dass das Formwerkzeug 20 geschlossen ist und durch Beaufschlagung mit der inhomogenen Temperaturverteilung seitens der Heizeinrichtungen 22a, 22b, gesteuert durch die Steuerung 24, der Implantatkörper 8 des Implantats 10 entstanden ist, der nach Öffnen des Formwerkzeugs 20 aus diesem entformt werden kann.

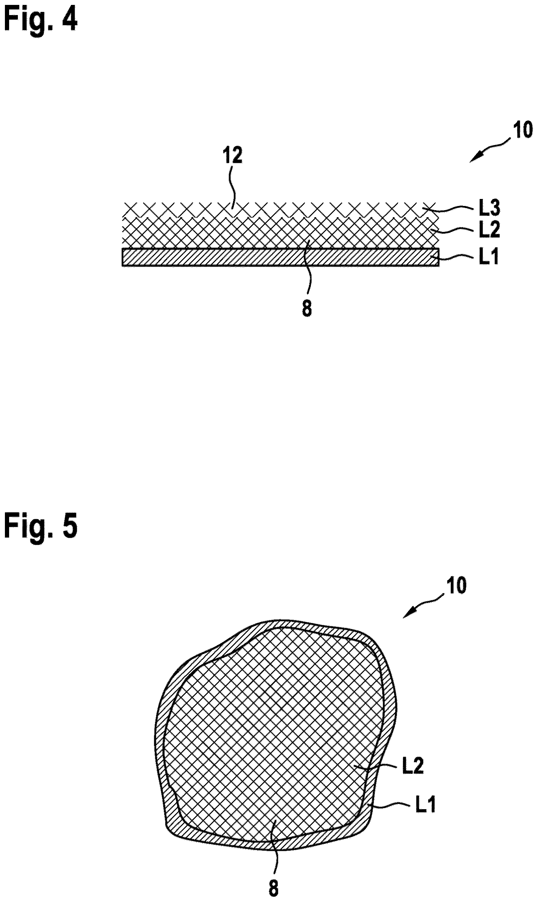

Fig. 4 zeigt eine geschnittene Seitenansicht eines Implantatkörpers mit einer Mehrzahl von Schichten und unterschiedlich porös ausgebildeten Teilabschnitten.

In der Fig. 4 ist ein Implantatkörper 8, der eine Mehrzahl von Schichten L1, L2, L3 aufweist, von denen die für den Betrachter unterste Schicht L1 massiv und damit ohne Porosität ausgebildet ist. Vorliegend bildet diese Schicht L1 eine den Implantatkörper 8 zumindest teilweise begrenzende Außenschicht, und unterscheidet sich strukturell von weiteren, innen liegenden Schichten L2, L3 unterscheidet. In Richtung für den Betrachter nach oben folgen diese weiteren Schichten L2, L3 mit unterschiedlicher Porosität, die Schicht L2 mit einer Porosität im Bereich von 10-20%, die Schicht L3 mit einer Porosität im Bereich von 20-40%. Hierdurch bilden die Schichten L2 und L3 poröse Teilabschnitte 12, 13 des Implantatkörpers 8 unterschiedlicher Porosität und auf diese Weise einen Porositätsgradienten.

Fig. 5 zeigt eine geschnittene Seitenansicht eines Implantatkörpers mit massiver Außenschicht.

Der Implantatkörper 8 weist einen porösen Außenbereich als ersten Teilabschnitt 13 auf, der mit einer geringeren Porosität als derjenigen eines porösen Innenbereichs als zweitem Teilabschnitt 12 ausgebildet ist.

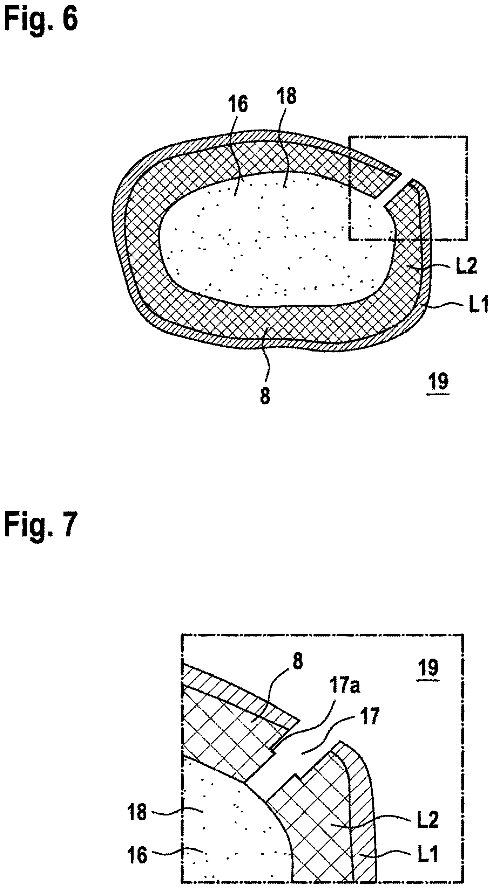

Fig. 6 zeigt eine geschnittene Seitenansicht eines Implantatkörpers in dessen Struktur ein Hohlraum ausgebildet ist.

In der Fig. 6 erkennt man einen Querschnitt eines Implantatkörpers 8, der wiederum einen porösen Außenbereich als einen Teilabschnitt 13 aufweist, der einen anderen Teilabschnitt 12 als Innenbereich mit höherer Porosität umschließt. Innerhalb seiner Struktur ist der Implantatkörper 8 mit einem Hohlraum 16 versehen, der einen ovalen Querschnitt hat. In dem Hohlraum ist eine bioaktive Substanz 18 in Art eines Hydrogels aufgenommen, welches die Geweberegeneration fördert. Der Hohlraum 16 hat dabei eine Querausdehnung von einigen Millimetern.

Fig. 7 zeigt eine geschnittene Seitenansicht eines Teilausschnitt der Gestaltung gemäß Fig. 6.

In dem Ausschnitt der Fig. 7 ist gezeigt, dass der Hohlraum 16 des Implantatkörpers 8 mit einem Schnittstellenabschnitt 17 versehen ist, der den Hohlraum 16 mit dem Außenraum 19 des Implantats 10 verbindet. Hierdurch ist ein Zugang zu dem Hohlraum 16 geschaffen, über den die bioaktive Substanz 18 zu einem gewünschten Zeitpunkt in dem Hohlraum deponiert werden kann. Ebenfalls gezeigt ist, dass an dem Schnittstellenabschnitt 17 ein Führungsmittel 17a angeordnet ist, die als flanschartige Querschnittseinschnürung des Schnittstellenabschnitts die Anlage, Aufnahme oder Ausrichtung eines nicht gezeigten Injektionsmittels, etwa eine Kanüle oder dergleichen, gestattet.

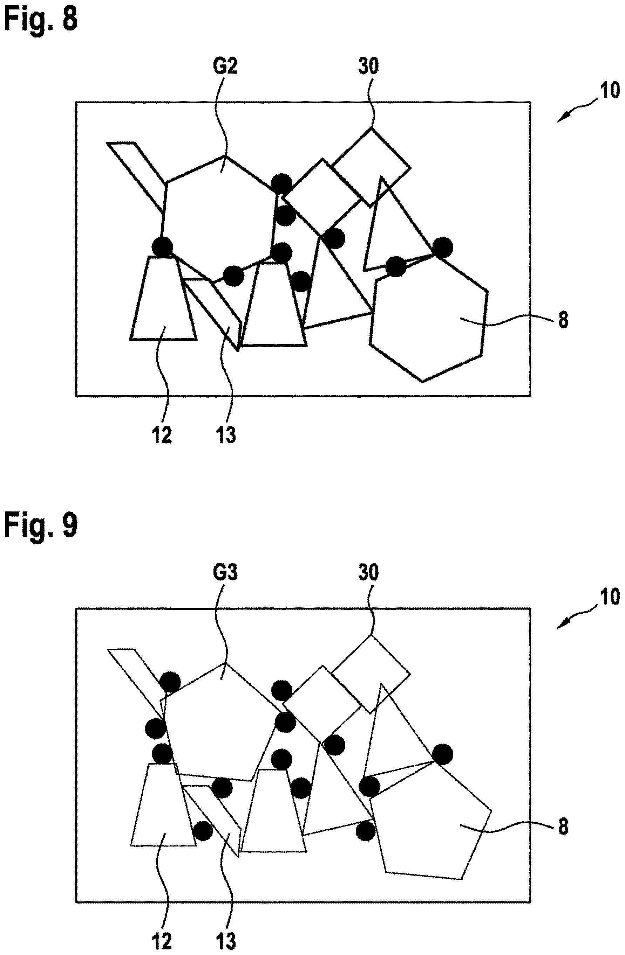

Fig. 8 zeigt eine geschnittene Seitenansicht einer Gestaltung des Implantatkörpers des Implantates mit poröser Oberfläche und porösen Teilabschnitten aus einem sechseckigen Granulat mit Beimischungen, und Fig. 9 zeigt eine geschnittene Seitenansicht einer Gestaltung des Implantatkörpers des Implantates mit poröser Oberfläche und porösen Teilabschnitten aus einem fünfeckigen Granulat mit Beimischungen.

Gemäß der Fig. 8 wurde ein sechseckiges Polyetheretherketon-(PEEK)-Granulat G2, und gemäß der Fig. 9 ein fünfeckiges PEEK-Granulat G3 eingesetzt.

Außerdem sind den Granulaten vor dem thermischen Verbinden jeweils unterschiedliche Zusatzstoffe beigemischt worden, bei dem Implantatkörper 8 der Fig. 8 eine keramische Komponente, und bei dem Implantatkörper 8 der Fig. 9 Silber und Kupfer als Metalle.

Wenn die Pulver zur Herstellung der Granulate jeweils eine entsprechende Vorbehandlung erfahren haben, sind die entstandenen Porenstrukturen jeweils intrinsisch bioaktiv, vorliegend antimikrobiell aktiv. Beide Implantatkörper 8 weisen dabei eine poröse Oberfläche 30 auf, sind also, wie bereits erwähnt, offenporige Strukturen. Sie bilden dabei mit ihren porösen Teilabschnitten 12, 13 interkonnektierende Porenstrukturen mit Poren in der Größenordnung einiger 100 µm, die das Einwachsen biologischen Gewebes unterstützen und die Festigkeit des Implantates 10 erhöhen. Das Implantat 10 wird dabei primär spezifisch über seine porösen Eigenschaften sowie über die makrostrukturellen Eigenschaften seines Herstellungsprozesses fixiert.

## Patentansprüche

1. Verfahren zur Herstellung eines biokompatiblen Implantates (10) mit den folgenden Schritten:
S1) Bereitstellen eines Kunststoff-Pulvers (2) aus einem Polyaryletherketon;
S2) Erhitzen und Verpressen des Kunststoff-Pulvers (2) unter Ausbildung zumindest eines Zwischenstücks (4);
S3) mechanisches Zerkleinern des zumindest einen Zwischenstücks (4) zu einem Granulat (G1, G2, G3); und
S4) thermisches Verbinden des Granulats (G1, G2, G3) in einem Formwerkzeug (20) zu einem Implantat (10), wobei das wenigstens eine Granulat (G1, G2, G3) beim thermischen Verbinden mit einer räumlich inhomogenen Wärmeverteilung beaufschlagt wird, wobei das Implantat (10 einen Implantatkörper (8) mit wenigstens einem ersten porös ausgebildeten Teilabschnitt (12) und einem zweiten porös ausgebildeten Teilabschnitt (13) aufweist, wobei sich der erste poröse Teilabschnitt (12) und der zweite poröse Teilabschnitt hinsichtlich ihrer Porosität unterscheiden.

2. Verfahren nach Anspruch 1, wobei das Formwerkzeug (20) mit einer Mehrzahl von Heizeinrichtungen (22a, 22b) beheizt wird, die beim thermischen Verbinden des Granulats (G1, G2, G3) jeweils separat angesteuert werden, um die inhomogene Wärmeverteilung zu erzeugen.

3. Verfahren nach Anspruch 1 oder 2, wobei das Polyaryletherketon (2) Polyetheretherketon ist.

4. Verfahren nach einem der vorhergehenden Ansprüche, wobei das Granulat (G1, G2, G3) Partikel mit einem runden, einem abgerundeten, einem teilweise eckigen oder einem mehreckigen Querschnitt aufweist.

5. Verfahren nach einem der vorhergehenden Ansprüche, wobei das Granulat (G1, G2, G3) Partikel mit einem runden Querschnitt aufweist.

6. Verfahren nach einem der vorhergehenden Ansprüche, wobei vor der Herstellung des Granulats (G1, G2, G3) das Kunststoff-Pulver (2) einer antimikrobiellen Behandlung unterzogen wird und/oder dem Kunststoff-Pulver (2) eine bioaktive Komponente beigemischt wird.

7. Verfahren nach einem der vorhergehenden Ansprüche, wobei das Granulat (G1, G2, G3) eine Partikelgröße zwischen 1 µm und 3000 µm aufweist.

8. Verfahren nach einem der vorhergehenden Ansprüche, wobei das Formwerkzeug (20) vor dem thermischen Verbinden des Granulats (G1, G2, G3) mit einer Mehrzahl sich hinsichtlich ihrer Partikelgröße und/oder -form unterscheidender Granulate (G1, G2, G3) befüllt wird.

9. Verfahren nach einem der vorhergehenden Ansprüche, wobei während des thermischen Verbindens des Granulats (G1, G2, G3) seitens des Formwerkzeugs (20) ein vorgebbarer Druck auf das wenigstens eine Granulat (G1, G2, G3) ausgeübt wird.

10. Verfahren nach einem der vorhergehenden Ansprüche, wobei das Formwerkzeug (20) ein Formgebungsmittel aufweist, durch welches beim thermischen Verbinden des Granulats (8) innerhalb des Implantatkörpers (8) ein Hohlraum (16) ausgebildet wird.

## Claims

1. Method for producing a biocompatible implant (10), comprising the following steps:
S1) providing a plastics powder (2) composed of a polyaryletherketone;
S2) heating and pressing the plastics powder (2) to form at least one intermediate piece (4);
S3) mechanically comminuting the at least one intermediate piece (4) to form a granular material (G1, G2, G3); and
S4) thermally bonding the granular material (G1, G2, G3) in a mould (20) to form an implant (10), wherein the at least one granular material (G1, G2, G3) is exposed to a spatially inhomogeneous heat distribution during the thermal bonding, wherein the implant (10) has an implant body (8) having at least one first porous section (12) and one second porous section (13), wherein the first porous section (12) and the second porous section differ in their porosity.

2. Method according to Claim 1, wherein the mould (20) is heated with a plurality of heating devices (22a, 22b) which are each activated separately during the thermal bonding of the granular material (G1, G2, G3) in order to produce the inhomogeneous heat distribution.

3. Method according to Claim 1 or 2, wherein the polyaryletherketone (2) is polyetheretherketone.

4. Method according to any of the preceding claims, wherein the granular material (G1, G2, G3) comprises particles having a round, rounded, partially angular or polygonal cross section.

5. Method according to any of the preceding claims, wherein the granular material (G1, G2, G3) comprises particles having a round cross section.

6. Method according to any of the preceding claims, wherein, before the preparation of the granular material (G1, G2, G3), the plastics powder (2) is subjected to an antimicrobial treatment and/or a bioactive component is admixed in the plastics powder (2) .

7. Method according to any of the preceding claims, wherein the granular material (G1, G2, G3) has a particle size of between 1 um and 3000 µm.

8. Method according to any of the preceding claims, wherein the mould (20) is filled with a plurality of granular materials (G1, G2, G3) of differing particle size and/or particle shape before the thermal bonding of the granular material (G1, G2, G3).

9. Method according to any of the preceding claims, wherein the mould (20) exerts a specifiable pressure on the at least one granular material (G1, G2, G3) during the thermal bonding of the granular material (G1, G2, G3).

10. Method according to any of the preceding claims, wherein the mould (20) comprises a shaping means which forms a cavity (16) within the implant body (8) during the thermal bonding of the granular material (8).

## Revendications

1. Procédé de fabrication d'un implant biocompatible (10), ledit procédé comprenant les étapes suivantes :
51) fournir une poudre de matière synthétique (2) de polyaryléthercétone ;
S2) chauffer et presser la poudre de matière synthétique (2) pour former au moins une pièce intermédiaire (4) ;
S3) broyer mécaniquement l'au moins une pièce intermédiaire (4) pour obtenir un granulat (G1, G2, G3) ; et
S4) lier thermiquement le granulat (G1, G2, G3) dans un outil de moulage (20) pour obtenir un implant (10), l'au moins un granulat (G1, G2, G3) étant soumis à une répartition de chaleur spatialement inhomogène lors de la liaison thermique, l'implant (10) comportant un corps d'implant (8) pourvu d'au moins une première portion poreuse (12) et d'une deuxième portion poreuse (13), la première portion poreuse (12) et la deuxième portion poreuse différant en termes de porosité.

2. Procédé selon la revendication 1, l'outil de moulage (20) étant chauffé avec une pluralité de dispositifs de chauffage (22a, 22b) qui sont chacun commandés séparément pendant la liaison thermique du granulat (G1, G2, G3) afin de générer la distribution de chaleur inhomogène.

3. Procédé selon la revendication 1 ou 2, la polyaryléthercétone (2) étant la polyétheréthercétone.

4. Procédé selon l'une des revendications précédentes, le granulat (G1, G2, G3) comportant des particules ayant une section transversale ronde, arrondie, partiellement angulaire ou polygonale.

5. Procédé selon l'une des revendications précédentes, le granulat (G1, G2, G3) comportant des particules ayant une section transversale ronde.

6. Procédé selon l'une des revendications précédentes, la poudre de matière synthétique (2) étant soumise, avant la fabrication du granulat (G1, G2, G3), à un traitement antimicrobien et/ou un composant bioactif étant ajouté à la poudre de matière synthétique (2).

7. Procédé selon l'une des revendications précédentes, le granulat (G1, G2, G3) ayant une granulométrie comprise entre 1 µm et 3000 µm.

8. Procédé selon l'une des revendications précédentes, l'outil de moulage (20) étant rempli, avant la liaison thermique du granulat (G1, G2, G3), d'une pluralité de granulats (G1, G2, G3) qui diffèrent en termes de taille et/ou de forme de particule.

9. Procédé selon l'une des revendications précédentes, une pression spécifiable étant exercée, pendant la liaison thermique du granulat (G1, G2, G3), sur l'au moins un granulat (G1, G2, G3) par l'outil de moulage (20).

10. Procédé selon l'une des revendications précédentes, l'outil de moulage (20) comportant un moyen de formage à travers lequel permettant de ménager une cavité (16) à l'intérieur du corps d'implant (8) lors de la liaison thermique du granulat (8).
